(19) **Europäisches Patentamt / European Patent Office / Office européen des brevets**

(11) **EP 3 477 292 B1**

(12) **EUROPEAN PATENT SPECIFICATION**

(45) Date of publication and mention
of the grant of the patent:
**13.05.2020 Bulletin 2020/20**

(51) Int Cl.:
*G01N 27/447* (2006.01)  *G01N 33/487* (2006.01)
*G01N 33/49* (2006.01)

(21) Application number: **18201557.8**

(22) Date of filing: **19.10.2018**

(54) **CAPILLARY ELECTROPHORESIS ANALYSIS METHOD**

ANALYSEVERFAHREN ZUR KAPILLARELEKTROPHORESE

PROCÉDÉ D'ANALYSE PAR ÉLECTROPHORÈSE CAPILLAIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **23.10.2017 JP 2017204709**

(43) Date of publication of application:
**01.05.2019 Bulletin 2019/18**

(73) Proprietor: **ARKRAY, Inc.
Kyoto-shi, Kyoto 601-8045 (JP)**

(72) Inventors:
• **KAWANO, Masao
Kyoto-shi, Kyoto 602-0008 (JP)**
• **ONUMA, Naotsugu
Kyoto-shi, Kyoto 602-0008 (JP)**

(74) Representative: **Dehns
St. Bride's House
10 Salisbury Square
London EC4Y 8JD (GB)**

(56) References cited:
EP-A1- 2 993 467  EP-A1- 3 315 959
JP-A- 2018 072 336  US-A1- 2010 155 241
US-B2- 7 223 325

• **RENATA MAYUMI SAITO ET AL: "Monitoring the Electroosmotic Flow in Capillary Electrophoresis Using Contactless Conductivity Detection and Thermal Marks", ANALYTICAL CHEMISTRY, vol. 79, no. 1, 1 January 2007 (2007-01-01), pages 215-223, XP055568004, US ISSN: 0003-2700, DOI: 10.1021/ac0615293**

**Description**

Technical Field

The present application relates to an analysis method, and in particular relates to an analysis method for analyzing a sample using capillary electrophoresis.

Related Art

Various types of proteins are analyzed as indicators indicating a condition of a living body. In particular, hemoglobin (Hb) blood cells contains several types of hemoglobin, including normal hemoglobin (HbA) as well as plural types of hemoglobin variants (HbC, HbD, HbE, HbS, etc.).

Component analysis (such as a method using liquid chromatography or the like) of a sample is performed, for example, by measuring a sample of a known composition in advance to confirm a detection time at which a desired component is detected and a peak profile thereof the component, which are compared to an analysis result of a specimen to identify the component (cf., e.g. JP S60-73458 A).

When analyzing the types of hemoglobin (as components) in blood (as a sample), detection times differ depending on the components, and this fact is utilized to perform separation analysis. In separation analysis, variability in each measurement (differences in detection time due to column deterioration, state of eluent storage, or the like) is taken into account when, for example, a particular component is identified as being present when the detection time of that particular component falls in a predetermined detection time range.

Electrophoresis techniques are employed as one option for techniques to analyze hemoglobin (cf., e.g. JP H11-337521 A). Sample analysis using an electrophoresis technique is performed in a state in which an analysis chip is loaded into an analysis device. The analysis chip holds a sample and provides a place for the target sample to be analyzed. The analysis chip may be a disposable analysis chip intended for disposal after completing only a single analysis. A method for analyzing hemoglobin using such a disposable analysis chip is disclosed in, for example, JP 2016-57289 A.

With disposable analysis chips, even when analyzing the same sample, difference of the analysis chips either between different lots or in an identical lot causes the detection time of specific components to fluctuate. With analysis chips intended for repeated use, the accuracy of analysis results can be improved by performing corrections based on comparison with known analysis results. However, with disposable analysis chips, situations may arise in which accurate analysis results cannot be obtained and components are incorrectly identified when significant error due to fluctuations in detection time arises due to individual differences in analysis chips.

SUMMARY

In consideration of the above circumstances, at least preferred embodiments of the present invention may provide an analysis method suitable to improving analysis accuracy in analysis of samples performed by employing a disposable analysis chip.

An analysis method provided according to the present invention is an analysis method for analyzing a sample using capillary electrophoresis by applying a voltage to a sample solution introduced to a micro flow path, performing separation analysis for a component contained in the sample solution, and measuring an optical measurement value at a measurement section of the micro flow path corresponding to an elapsed time after a start of measurement, the analysis method comprising: a waveform forming step of forming a waveform related to the optical measurement value corresponding to the elapsed time since the start of measurement; an interface detection step of determining an interface detection time when an interface between the sample solution and a migration liquid reaches a predetermined measurement position in the micro flow path, based on the optical measurement value; a waveform peak specification step of specifying waveform peaks appearing in the waveform and of identifying a maximum waveform peak which is the largest waveform peak out of the waveform peaks; and characterized by a component identification step of identifying a component contained in the sample solution, said component corresponding to the maximum waveform peak, based on a maximum-peak detection time which is the elapsed time corresponding to the maximum waveform peak, as well as on the interface detection time.

"A predetermined measurement position in the micro flow path" means a position through which a measurement light passes to measure the optical measurement value. Note that an optical measurement value related to this interface may be measured at the same position as the measurement section for measuring the optical measurement value related to the component to be identified, or may be measured at a different position.

Preferably, in the interface detection step, the interface detection time is determined based on a change in the optical measurement value when the interface reaches the predetermined measurement position.

Preferably, the optical measurement value is an optical absorbance.

Preferably, in the component identification step, a component corresponding to the maximum waveform peak is identified based on a comparison of the maximum-peak detection time to the interface detection time.

Preferably, in the component identification step, components corresponding to the maximum waveform peak and a second waveform peak are identified based on a comparison between the maximum-peak detection time, a second-peak detection time, which is the elapsed time corresponding to the second waveform peak configuring the second largest waveform peak out of the waveform peaks specified in the waveform peak specification step, and the interface detection time.

Preferably, in the component identification step, components corresponding to the maximum waveform peak and the second waveform peak are identified based on a ratio of a difference between the maximum-peak detection time and the interface detection time, to a difference between the second-peak detection time and the interface detection time.

Note that the sample solution of the above analysis method is a solution containing a sample to be analyzed, and this definition encompasses both sample solutions that are 100% neat sample, and sample solutions in which the sample has been appropriately diluted. The sample solution is not particularly limited as long as arrival of the interface can be identified, and as long as the sample solution has a liquid form. Examples of such liquids include diluents having a solid form of the sample, e.g. suspended, dispersed, or dissolved in a liquid medium. When the sample is a liquid, for example, the neat liquid of the sample may be used as-is as the sample solution. In cases in which the concentration of the neat liquid is too high, a diluent having the neat liquid suspended, dispersed, or dissolved in a liquid medium may be employed use as the sample solution. There are no particular limitations to the liquid medium as long as the liquid medium is capable of suspending, dispersing, or dissolving the sample, and examples of the liquid medium include water or a buffer solution. Examples of the sample include, e.g. a specimen derived from a biological body, a specimen derived from the environment, a metal, a chemical substance, a drug, or the like. The specimen derived from a biological body is not particularly limited, and examples thereof include urine, blood, hair, saliva, sweat, nails and the like. The blood specimen may be, for example, erythrocytes, whole blood, serum, blood plasma, or the like. Examples of the biological body include a human, a non-human animal, a plant, or the like, and the non-human animal may be, for example, a mammal other than a human, a reptile, an amphibian, a fish, an insect, or the like. The specimen derived from the environment is not particularly limited, and examples thereof include a food product, water, soil, the atmosphere, an air sample, or the like. Examples of the food include, for example, fresh food products, and processed food products. Examples of the water include, for example, drinking water, underground water, river water, sea water, household effluent, and the like.

Preferably, the sample solution is a solution containing blood as a sample, and the component is hemoglobin.

Preferably, a disposable analysis chip that includes the micro flow path is employed.

At least preferred embodiments of the invention may provide an analysis method that is suitable for improving analysis accuracy in analysis of samples using a disposable analysis chip.

BRIEF DESCRIPTION OF THE DRAWINGS

The embodiments will be described in detail by way of example only and with reference to the following figures, in which:

Fig. 1 is a schematic view of an analysis system capable of being employed to execute an analysis method according to an embodiment of the present application;
Fig. 2 is a plan view illustrating an analysis chip employed in the analysis system of Fig. 1;
Fig. 3 is a cross-section taken along line III-III in Fig. 2;
Fig. 4 is a flowchart illustrating an analysis method according to an embodiment of the present application;
Fig. 5 is a flowchart illustrating procedures in a preparation step;
Fig. 6 is a cross-section illustrating one procedure of the preparation step of Fig. 5;
Fig. 7 is a cross-section illustrating one procedure of the preparation step of Fig. 5;
Fig. 8 is a cross-section illustrating one procedure of the preparation step of Fig. 5;
Fig. 9 is a cross-section illustrating one procedure of the preparation step of Fig. 5;
Fig. 10 is a flowchart illustrating procedures in an analysis step;
Fig. 11 illustrates an example of an electropherogram of an HbAA specimen;
Fig. 12 illustrates an example of an electropherogram of an HbCC specimen;
Fig. 13 illustrates an example of an electropherogram of an HbEE specimen;
Fig. 14 illustrates an example of an electropherogram of an HbSS specimen;
Fig. 15 illustrates an example of an electropherogram of an HbSC specimen; and
Fig. 16 illustrates the electropherograms of Fig. 11 to Fig. 15 overlaid together.

DETAILED DESCRIPTION

Detailed explanation follows regarding a preferable embodiment of the present application, with reference to the drawings.

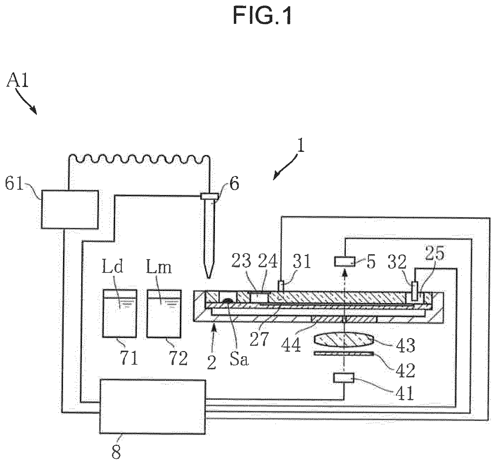

Fig. 1 illustrates a schematic configuration of an example of an analysis system A1 that can be employed to perform an analysis method according to an embodiment of the present application. The analysis system A1 comprises an analysis device 1 and an analysis chip 2. The analysis system A1 is a system to execute a method for analyzing a sample Sa using capillary electrophoresis. The sample Sa is not particularly limited, and in the present embodiment, explanation will be given using blood collected from a human body as an example of the sample Sa. Components to be analyzed are defined as those components which are subject to analysis from out of the components contained in the sample Sa.

Examples of such components to be analyzed include hemoglobin (Hb), albumin (Alb), globulin ($\alpha 1$, $\alpha 2$, $\beta$, and $\gamma$-globulin), fibrinogen, or the like. Examples of the hemoglobin referred to above include several types of hemoglobin, such as normal hemoglobin (HbA), hemoglobin variants (HbC, HbD, HbE, HbS, etc.), fetal hemoglobin (HbF), and the like. Hemoglobin variants are known to cause various types of diseases and conditions (e.g. HbS causes sickle cell anemia), and so identifying hemoglobin types is expected to be helpful in diagnosing and treating diseases or conditions. In the following explanation, explanation will be given of an example of a case in which the components to be analyzed are hemoglobins (in particular, types of hemoglobin variants). Normal adult hemoglobin is predominantly constituted by HbA, with only small amounts of HbF and HbA2 contained therein. Hemoglobin is a tetramer, and, in the case of HbA, for example, is configured by two $\alpha$ chains and two $\beta$ chains. A mutation in the genetic sequence responsible for the production of either the $\alpha$ chains or the $\beta$ chains may cause a different amino acid sequence in the produced chain or may suppress the corresponding chain to be produced, resulting in the production of a different type of hemoglobin from a normal one. Generally, such a type of hemoglobin is referred to as a hemoglobin variant. The genotype for hemoglobin of a normal person is HbA/HbA homozygous. However, the genotype of a carrier of a hemoglobin variant is either HbA/HbV heterozygous (where HbV is a hemoglobin variant other than HbA) or HbV/HbV homozygous (which may be heterozygous in cases in which the HbVs are different from each other). In clinical laboratory testing, a blood specimen sourced from a normal person is referred to as an HbAA specimen, a blood specimen sourced from a person who is HbA/HbV heterozygous is referred to as an HbAV specimen (where V is a given variant), and a blood specimen sourced from a person who is HbV/HbV homozygous (or heterozygous in the case in which the HbV are different from each other) is referred to as an HbVV specimen (where each V is a given variant). Thus, an HbAS specimen, for example, is predominantly constituted by HbA and HbS, with only small amounts of HbF and HbA2 contained therein. An HbSS specimen, for example, is predominantly constituted by HbS, with only small amounts of HbF and HbA2 contained therein. Note that in the following explanation, the notation "HbXY" (X and Y each being one out of A, C, D, E, or S, and being different from each other) refers to an HbX/HbY heterozygous hemoglobin variant, with the former (namely, HbX) making up a larger proportion of the overall hemoglobin than the latter (namely, HbY).

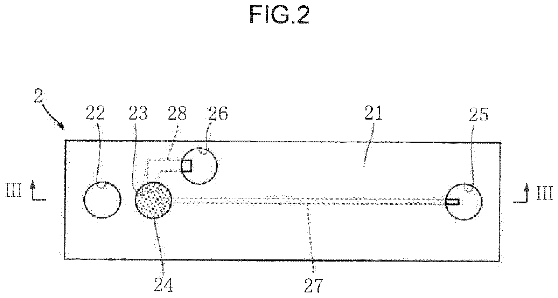

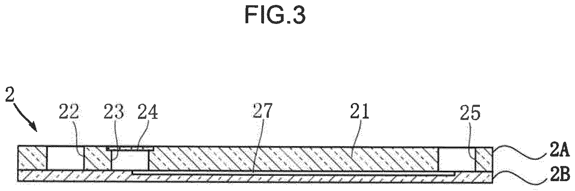

The analysis chip 2 holds the sample Sa, and provides a place for performing analysis on the target sample Sa in a state in which the analysis chip 2 is loaded in the analysis device 1. In the present embodiment, the analysis chip 2 is a so-called disposable analysis chip, which is meant to be disposed of after completion of a single analysis. As illustrated in Fig. 2 and Fig. 3, the analysis chip 2 includes a body 21, a mixing reservoir 22, an introduction reservoir 23, a filter 24, a waste reservoir 25, an electrode reservoir 26, a capillary channel 27, and a communication flow path 28. Fig. 2 is a plan view of the analysis chip 2, and Fig. 3 is a cross-section taken along III-III in Fig. 2. Note that the analysis chip 2 is not limited to being a disposable chip, and the analysis chip 2 may be a chip that can be employed several times for analysis. Further, the analysis system according to the present embodiment is not limited to a configuration in which the analysis chip 2 is provided as a separate body loaded in the analysis device 1. The analysis system according to the present application may be configured with a functional part that accomplishes similar functionality to that of the analysis chip 2 incorporated in the analysis device 1.

The body 21 is a stage for the analysis chip 2. The material of the body 21 is not particularly limited and examples thereof include glass, fused silica, plastic, and the like. In the present embodiment, the body 21 is formed from separate bodies of an upper portion 2A and a lower portion 2B illustrated in Fig. 3, which are joined to each other. Note that the body 21 is not limited to be configured as above, and for example, the body 21 may be formed as a single integrated unit.

The mixing reservoir 22 is an example of a site where a mixing step is performed to mix the sample Sa and a diluent Ld, described later. The mixing reservoir 22 is, for example, configured as a recess open to an upper side by a through hole formed in the upper portion 2A of the body 21. The introduction reservoir 23 is a reservoir for introducing a sample mixture Sm as a sample solution obtained from the mixing step in the mixing reservoir 22. The introduction reservoir 23 is, for example, configured as a recess open to the upper side by a through hole formed in the upper portion 2A of the body 21.

The filter 24 is provided at an opening of the introduction reservoir 23. The opening serves as an example of an introduction path to the introduction reservoir 23. The specific configuration of the filter 24 is not limited, and preferable examples of the configuration include Cellulose Acetate Membrane Filters (Advantec, 0.45 $\mu$m pore diameter).

The waste reservoir 25 is a reservoir that is positioned on a downstream side of an electroosmotic flow during electrophoresis. The waste reservoir 25 is, for example, configured as a recess open to the upper side by a through hole formed in the upper portion 2A of the body 21. The electrode reservoir 26 is a reservoir into which a first electrode 31 is inserted in an analysis step employing electrophoresis. The electrode reservoir 26 is, for example, configured as a recess open to the upper side by a through hole formed in the upper portion 2A of the body 21. The communication flow path 28 connects the introduction reservoir 23 and the electrode reservoir 26 together, and provides a connecting path between the introduction reservoir 23 and the electrode reservoir 26.

The capillary channel 27 is a micro flow path connecting the introduction reservoir 23 and the waste reservoir 25 together, and is a place where the electroosmotic flow (EOF) occurs during electrophoresis. The capillary channel 27 is, for example, configured as a groove formed in the lower portion 2B of the body 21. Note that recesses or the like may be formed in the body 21 as appropriate to promote irradiation of light onto the capillary channel 27 and to promote output of light that passes through the capillary channel 27. The size of the capillary channel 27 is not particularly limited. As an example, the capillary channel 27 has a width of from 25 $\mu$m to 100 $\mu$m, a depth of from 25 $\mu$m to 100 $\mu$m, and a length of from 5 mm to 150 mm. The overall size of the analysis chip 2 is appropriately set so as to accommodate the size of the capillary channel 27 and the size, placement, and so on of the mixing reservoir 22, the introduction reservoir 23, the waste reservoir 25, and the electrode reservoir 26.

Note that an analysis chip 2 with the above configuration is merely an example, and any analysis chip with a configuration that enables electrophoresis analysis to be performed may be appropriately employed therefor.

The analysis device 1 performs analysis processing on the sample Sa, in a state in which the analysis chip 2 spotted with the sample Sa is loaded in the analysis device 1. The analysis device 1 includes first and second electrodes 31, 32, a light source 41, an optical filter 42, a lens 43, a slit 44, a detector 5, a dispenser 6, a pump 61, a diluent reservoir 71, a migration liquid reservoir 72, and a control section 8. Note that the light source 41, the optical filter 42, the lens 43, and the detector 5 provide an example of what is referred to as a measurement section in the present embodiment.

The first electrode 31 and the second electrode 32 are for applying a predetermined voltage to the capillary channel 27 during electrophoresis. The first electrode 31 is inserted into the electrode reservoir 26 of the analysis chip 2, and the second electrode 32 is inserted into the waste reservoir 25 of the analysis chip 2. The voltage that is applied by the first electrode 31 and the second electrode 32 is not particularly limited, and may, for example, be from 0.5 kV to 20 kV.

The light source 41 is a portion that emits light to measure an optical absorbance as an optical measurement value during electrophoresis. The light source 41 is provided, for example, with an LED chip that emits light of a predetermined wavelength range. The optical filter 42 attenuates light of predetermined wavelengths in the light from the light source 41 and transmits light of other wavelengths therein. The lens 43 focuses light that is transmitted through the optical filter 42 onto an analysis site of the capillary channel 27 of the analysis chip 2. The slit 44 excludes excess light from the light focused by the lens 43 which might cause scattering and the like.

The detector 5 receives light from the light source 41 transmitted through the capillary channel 27 of the analysis chip 2, and includes a photodiode, a photo IC, and the like.

In this manner, the route along which the light emitted by the light source 41 reaches the detector 5 is referred to as a light path. An optical measurement value for the solution (namely, a sample solution, a migration liquid, or a solution containing a mixture of the two) flowing through the capillary channel 27 is measured at the position where the light path intersects the capillary channel 27. Namely, the position where the light path from the light source 41 to the detector 5 intersects the capillary channel 27 is referred to as the measurement section for the optical measurement value. The optical measurement value may, for example, represent optical absorbance. Optical absorbance represents a degree to which light in the light path is absorbed by the solution flowing through the capillary channel 27, and is expressed as an absolute value of a common logarithm of a ratio of an incident light intensity to a transmitted light intensity. In such cases, a generic spectrophotometer may be employed as the detector 5. Note that any optical measurement value other than the optical absorbance, such as the simple transmitted light intensity, may be employed in the present embodiment. The following explanation describes an example in which optical absorbance is employed as the optical measurement value.

The dispenser 6 dispenses desired amounts of the diluent Ld, the migration liquid Lm, and the sample mixture Sm, and the dispenser 6 includes a nozzle, for example. The dispenser 6 can be freely moved between plural predetermined positions in the analysis device 1 using an unillustrated drive mechanism. The pump 61 is a power supply for suction and purging for the dispenser 6. Further, the pump 61 may be employed as a power supply for suction and purging for unillustrated ports provided to the analysis device 1. Such ports may be employed to fill the migration liquid Lm or the like. Further, a dedicated pump may also be provided separate to the pump 61.

The diluent reservoir 71 is a reservoir for storing the diluent Ld. The diluent reservoir 71 may be a reservoir that is permanently installed to the analysis device 1, or may be a container that contains a predetermined amount of the diluent Ld and is loaded into the analysis device 1. The migration liquid reservoir 72 is a reservoir for storing the migration liquid Lm. The migration liquid reservoir 72 may be a reservoir that is permanently installed to the analysis device 1, or may be a container that contains a predetermined amount of the migration liquid Lm and is loaded into the analysis device 1.

The diluent Ld is mixed with the sample Sa to produce the sample mixture Sm as a sample solution. The main agent of the diluent Ld is not particularly limited, and examples include water and physiological saline. The diluent Ld is preferably a liquid including components similar to those of the migration liquid Lm, described later. Further, additives may be added as necessary to the dilu-

entent Ld in addition to the main agent.

The migration liquid Lm is filled into the waste reservoir 25 and the capillary channel 27 in an analysis step employing electrophoresis, and is a medium to generate electroosmotic flow therein during electrophoresis. Although the migration liquid Lm is not particularly limited, an acidic migration liquid is preferably used as the migration liquid Lm. The acid is, for example, citric acid, maleic acid, tartaric acid, succinic acid, fumaric acid, phthalic acid, malonic acid, or malic acid. Further, the migration liquid Lm preferably includes a weak base. The weak base is, for example, arginine, lysine, histidine, tris, or the like. The migration liquid Lm, for example, has a pH in the range of pH4.5 to pH6. The type of buffer of the migration liquid Lm is, for example, 2-(N-morpholino)ethanesulfonic acid (MES), N-(2-Acetamido)iminodiacetic acid (ADA), N-(2-Acetamido)-2-aminoethanesulfonic acid (ACES), N,N-Bis(2-hydroxyethyl)-2-aminoethanesulfonic acid (BES), 3-(N-morpholino)propanesulfonic acid (MOPS), N-Tris(hydroxymethyl)methyl-2-aminoethanesulfonic acid (TES), 2-[4-(2-hydroxyethyl)-1-piperazinyl]ethanesulfonic acid (HEPES), or the like. As stated above regarding the diluent Ld, additives may also be added to the migration liquid Lm as necessary.

Although examples of the migration liquid Lm, the diluent Ld, and the sample mixture Sm are given below, they may be freely selected in any combination with which a change in the optical measurement value is caused by the arrival at the interface between the sample solution (sample mixture Sm) and the migration liquid Lm during an interface detection time, described later.

Migration liquid Lm

The migration liquid Lm, for example, includes the following components:

Citric acid: 40 mM,
Chondroitin sulfate C sodium: 1.25% w/v,
Piperazine: 20 mM,
Polyoxyalkylene alkyl ether (Emulgen LS-110, Kao Corporation): 0.1% w/v,
Sodium azide: 0.02% w/v, and
ProClin 300: 0.025% w/v.

In addition to the above components, dimethylaminoethanol for pH regulation is dripped in to regulate to pH5.0.

Diluent Ld

The diluent Ld, for example, includes the following components:

Citric acid: 38 mM,
Chondroitin sulfate C sodium: 0.95% w/v,
1-(3-sulfopropyl) pyridinium hydroxide inner salt (NDSB-201): 475 mM,
2-(N-morpholino)ethanesulfonic acid (MES): 19mM,
Polyoxyalkylene alkyl ether (Emulgen LS-110, Kao Corporation): 0.4% w/v, Sodium azide: 0.02% w/v, and
ProClin 300: 0.025% w/v.

In addition to the above components, dimethylaminoethanol for pH regulation is dripped in to regulate to pH6.0.

Sample mixture Sm

The sample mixture Sm is prepared by adding 1.5 μL of the sample Sa to 60 μL of the diluent Ld.

The control section 8 controls each section of the analysis device 1. The control section 8 is, for example, provided with a CPU, a memory, an interface, and the like. The memory appropriately stores programs and various data for performing the analysis method according to the present embodiment, described later.

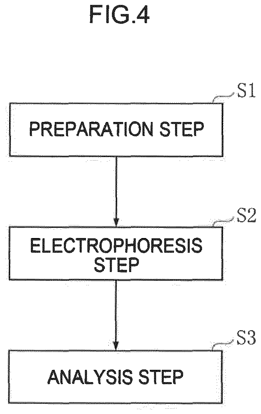

Next, explanation follows regarding an example of the analysis method according to the present embodiment performed employing the analysis system A1. Fig. 4 is a flowchart illustrating an analysis method of the present embodiment. The present analysis method includes a preparation step S1, an electrophoresis step S2, and an analysis step S3.

Preparation Step S1

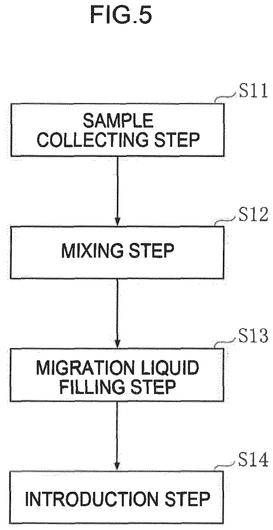

Fig. 5 is a flowchart illustrating a specific procedure for the preparation step S1. As illustrated in Fig. 5, the preparation step S1 of the present embodiment includes a sample collecting step S11, a mixing step S12, a migration liquid filling step S13, and an introduction step S14.

Sample Collecting Step S11

First, the sample Sa is prepared. In the present embodiment, the sample Sa is blood collected from a human body. The blood may be whole blood, fractionated blood, hemolyzed blood, or the like. Then, the analysis chip 2 is loaded into the analysis device 1 after the sample Sa is dispensed to the analysis chip 2.

Mixing Step S12

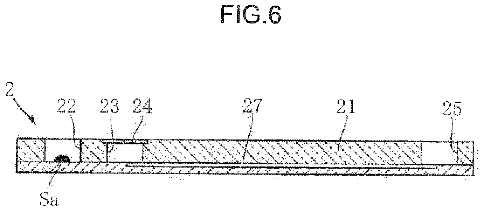

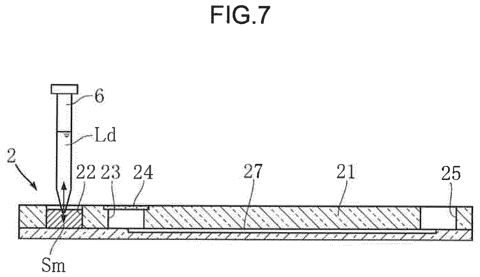

Next, the sample Sa and the diluent Ld are mixed together. Specifically, as illustrated in Fig. 6, a predetermined amount of the sample Sa has been spotted into the mixing reservoir 22 of the analysis chip 2. Next, a predetermined amount of the diluent Ld in the diluent reservoir 71 is drawn by the dispenser 6, and, as illustrated in Fig. 7, the predetermined amount of the diluent Ld is dispensed into the mixing reservoir 22 of the analysis chip 2. Then, the diluent Ld is repeatedly drawn and purged in the dispenser 6 by the pump 61 as the power supply for suction and purging. The sample Sa and the diluent Ld are thus mixed together in the mixing reservoir 22, and the sample mixture Sm as a sample solution is thereby obtained. The mixture of the sample Sa and the diluent Ld may be performed using other methods than drawing and purging with the dispenser 6.

Migration Liquid Filling Step S13

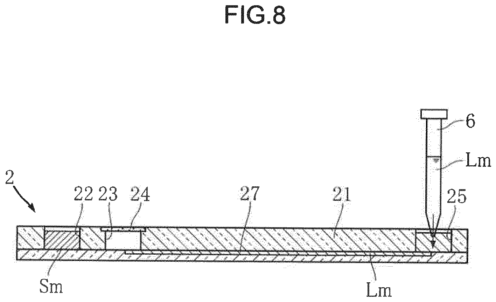

Next, the dispenser 6 is used to draw a predetermined amount of the migration liquid Lm from the migration liquid reservoir 72, and, as illustrated in Fig. 8, to dispense the predetermined amount of the migration liquid Lm into the waste reservoir 25 of the analysis chip 2. Then, the migration liquid Lm is filled into the waste reservoir 25 and the capillary channel 27 by, for example, appropriately drawing and purging through the port as described above.

Introduction Step S14

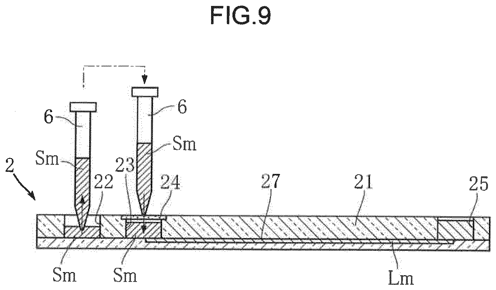

Next, as illustrated in Fig. 9, a predetermined amount of the sample mixture Sm is collected from the mixing reservoir 22 using the dispenser 6. Then, the predetermined amount of sample mixture Sm is introduced from the dispenser 6 to the introduction reservoir 23. When being introduced, the sample mixture Sm passes through the filter 24 provided at the opening of the introduction reservoir 23 as an example of an introduction path to the introduction reservoir 23. Further, in the present embodiment, the sample mixture Sm passes from the introduction reservoir 23, through the communication flow path 28, and fills the electrode reservoir 26. Then, the sample mixture Sm is caused to flow from the introduction reservoir 23 to the electrode reservoir 26 through the communication flow path 28. However, from the introduction reservoir 23 to the communication flow path 28, the sample mixture Sm flows in a direction substantially orthogonal to the length direction of the capillary channel 27 (see Fig. 2). On the other hand, the migration liquid Lm in the capillary channel 27 basically hardly moves at this stage. As a result, a clear interface arises between the sample mixture Sm and the migration liquid Lm due to shear flow occurring in the section connecting the introduction reservoir 23 to the capillary channel 27 (see Fig. 3). Note that any other methods, such as a physical provision of the movable filter at the boundary between the introduction reservoir 23 and the capillary channel 27, and a control for changing the method of flowing, may be employed as long as such an interface between the sample mixture Sm and the migration liquid Lm can arise by the methods.

Electrophoresis Step S2

Next, as illustrated in Fig. 1, the first electrode 31 is inserted into the electrode reservoir 26, and the second electrode 32 is inserted into the waste reservoir 25. Then, voltage is applied to the first electrode 31 and the second electrode 32 under instruction from the control section 8. This voltage is, for example, from 0.5 kV to 20 kV. Thus, electroosmotic flow is induced, causing the sample mixture Sm to gradually move from the introduction reservoir 23 to the waste reservoir 25 through the capillary channel 27. Meanwhile, since the sample mixture Sm is filled in the introduction reservoir 23, hemoglobin (Hb) as the components to be analyzed is electrophoresed in a state in which the sample mixture Sm in is continuously supplied the capillary channel 27. Meanwhile, the sample mixture Sm migrates through the capillary channel 27 while pushing the migration liquid Lm in the downstream direction, the interface between the sample mixture Sm and the migration liquid Lm being maintained. Further, the light source 41 starts to emit light, and the detector 5 measures optical absorbances. Then, the relationship is measured between the elapsed time since the start of voltage application by the first electrode 31 and the second electrode 32, and optical absorbance.

Analysis Step S3

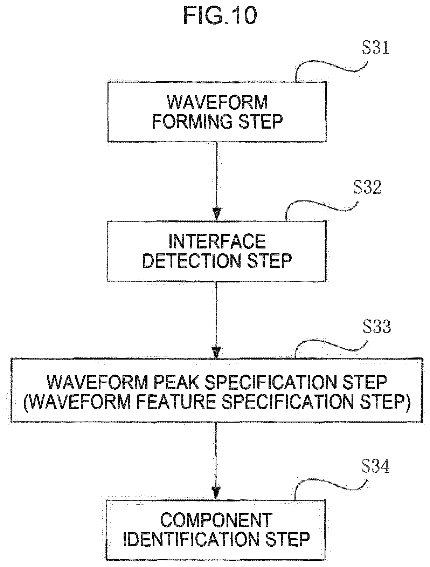

Optical absorbance peaks corresponding to components that have a comparatively fast moving rate in the sample mixture Sm appear at a comparatively earlier elapsed time since the start of voltage application. On the other hand, optical absorbance peaks corresponding to components that have a comparatively slow moving rate in the sample mixture Sm appear at a comparatively later elapsed time since the start of voltage application. Thereby, analysis (i.e., separation and measurement) of the components in the sample mixture Sm is thus performed. In the present embodiment, hemoglobin (especially, hemoglobin variants) contained in blood is analyzed, and the hemoglobin (hemoglobin variants) possibly contained in the sample mixture Sm (blood) is the component to be analyzed. Analysis step S3 is executed under control of the control section 8 based on the measured optical absorbance. As illustrated in Fig. 10, the analysis step S3 of the present embodiment includes a waveform forming step S31, an interface detection step S32, a waveform peak specification step S33, and a component identification step S34.

Waveform Forming Step S31

Since the measured optical absorbance described above is expressed as chronologically cumulative values from the time of the start of measurement, it is expressed as a monotonically increasing curve with time. In this step, the chronologically cumulative values of the measured optical absorbance are subjected to calculation processing (for example, differential processing (by taking the derivative with respect to time, i.e. the rate of change of the measured optical absorbance) or difference processing by the control section 8) to create an electropherogram. The voltage application start time (0 sec) is taken as a measurement start time, and a measurement waveform related to optical absorbance corresponding to the elapsed time (detection time) since the start of measurement is formed.

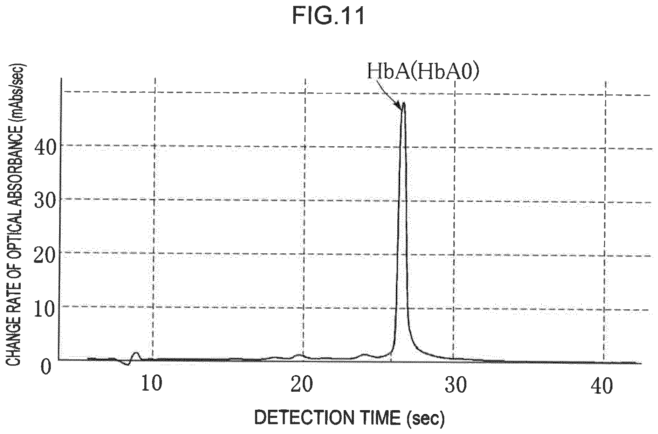

For example, Fig. 11 illustrates an electropherogram for an HbAA specimen that does not contain variant hemoglobin. The vertical axis represents differential values, which is a value obtained by performing calculation processing such that the measured optical absorbance is subjected to differential processing, and so the vertical axis is labeled "Change rate of optical absorbance (mAbs/sec)" (similar applies hereafter). In this specimen, the largest waveform peak (referred to hereafter as the "maximum waveform peak") arising due to HbA is clearly seen as a substantially single waveform peak. The detection time at which this waveform peak is obtained is a maximum-peak detection time.

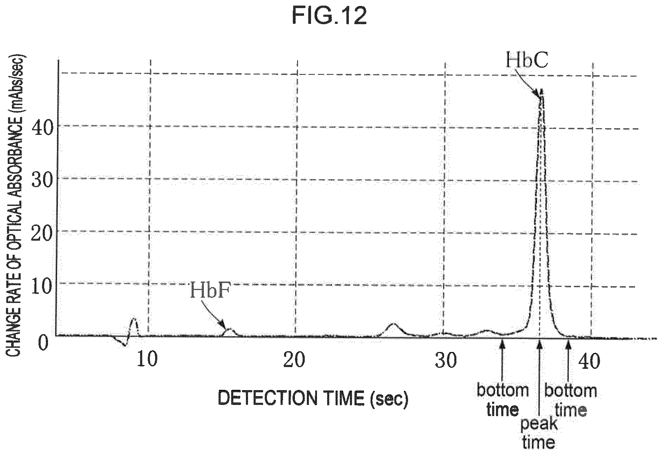

Fig. 12 illustrates an electropherogram for an HbCC specimen as an example of a homozygous hemoglobin variant. A maximum waveform peak arising due to HbC is clearly seen in this specimen. This waveform peak is detected at a detection time (maximum-peak detection time) later than the detection time of the waveform peak for HbA in Fig. 11.

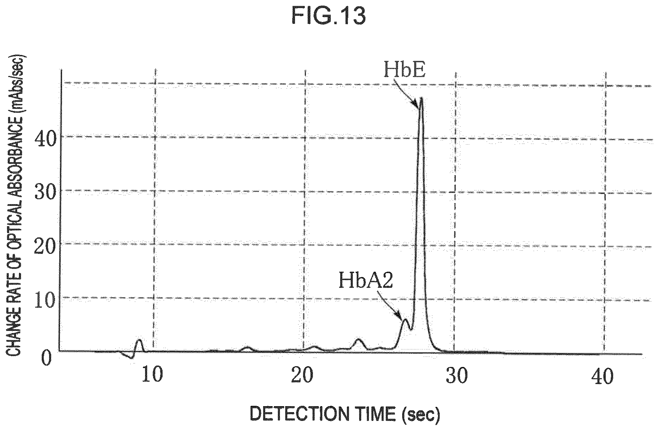

Fig. 13 illustrates an electropherogram for an HbEE specimen as an example of another homozygous hemoglobin variant. A maximum waveform peak arising due to HbE is clearly seen in this specimen. This waveform peak is detected at a detection time (maximum-peak detection time) that is slightly later than that of the waveform peak for HbA in Fig. 11, but is earlier than the detection time of the waveform peak for HbC in Fig. 12.

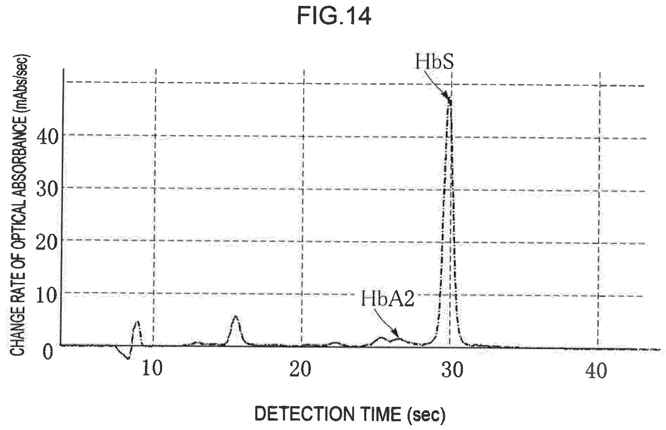

Fig. 14 illustrates an electropherogram for an HbSS specimen as an example of another homozygous hemoglobin variant. A maximum waveform peak arising due to HbS is clearly seen in this specimen. This waveform peak is detected at a detection time (maximum-peak detection time) that is slightly later than that of the waveform peak for HbE in Fig. 13, but is earlier than the detection time of the waveform peak for HbC in Fig. 12.

Although not illustrated in the drawings, in an HbDD specimen as a homozygous hemoglobin variant, a maximum waveform peak arising due to HbD is detected at a detection time (maximum-peak detection time) between the detection time for the waveform peak for HbE in Fig. 13 and the detection time for the waveform peak for HbS in Fig. 14.

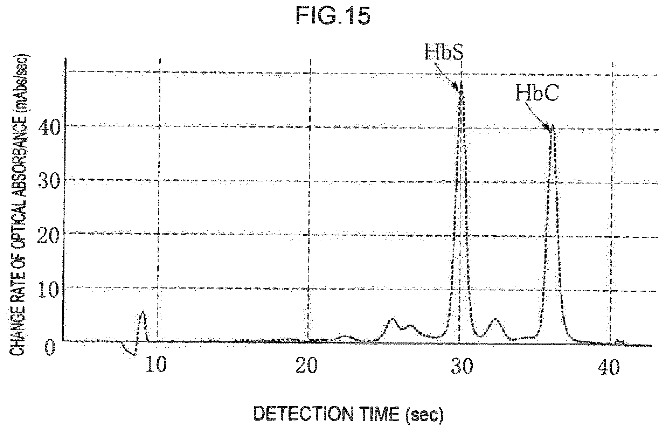

Fig. 15 illustrates an electropherogram for an HbSC specimen as an example of a heterozygous hemoglobin variant. In this specimen, a maximum waveform peak arising due to HbS is detected at substantially the same detection time as of the waveform peak illustrated in Fig. 14, which is a maximum-peak detection time. The second largest waveform peak (referred to hereafter as the "second waveform peak"), arising due to HbC, is detected at substantially the same detection time as of the waveform peak illustrated in Fig. 12, which is a second-peak detection time.

As described above, the measurement waveforms have waveform peak portions corresponding to the components contained in the sample mixture Sm (sample Sa), and then information related to the waveform peak portions is acquired. For each of the maximum waveform peak and the second waveform peak, the information relating to the waveform peak portion includes a peak time of a peak top portion (i.e. the detection time of the highest position of the waveform peak portion; see Fig. 12), a bottom time, or plurality of bottom times, for example two bottom times (i.e. the detection times of the lowest position of the waveform peak portion; see Fig. 12), and an area ratio of the waveform peak portion (i.e. the proportional area surrounded by the waveform peak portion between two bottoms on both sides of the peak top, with respect to the total area of the regions showing positive values as to change rate of optical absorbance in the measurement waveform). This information is, for example, obtained by calculation processing by the control section 8. Note that the bottoms may be determined using various methods. For example, the positions of minimum values appearing before and after the peak top in the immediate vicinity of the peak top may be taken as the bottoms. Alternatively, if such minimum values are not present (namely, if there is a monotonic decrease from the peak top), for example, the positions where the waveform reaches a predetermined base line as a reference for area calculations may be taken as the bottoms.

Interface Detection Step S32

As illustrated in Fig. 11 to Fig. 15, the small waveforms that can be seen approximately 10 seconds after the start of measurements, namely the waveforms where an initial dip is followed by an increase, indicate the initial detection of the electroosmotic flow (EOF) at the measurement section (specifically, the light path), which is an overall flow of liquid in the capillary occurring in accordance with voltage application during capillary electrophoresis. A time point at which the EOF is initially detected represents a time point indicating an arrival of an interface between the sample mixture Sm and the migration liquid Lm at the measurement section. In the present embodiment, a time from the start of measurement (where the time of the start of the measurement is the time that application of voltage to the sample mixture Sm begins) to this time point is referred to as the "interface detection time". In this step, the EOF is detected based on a change in optical absorbance as an optical measurement value, and the interface detection time is determined to be an elapsed time between the start of measurement and the detection of the EOF. Note that in cases in which a measurement section (i.e. a light path) exclusive for a measurement of the interface is provided separately from the measurement section and the light path, the interface detection time may be determined at the exclusive measurement section.

Waveform Peak Specification Step S33

In the waveform peak specification step S33 as a waveform feature specification step, a maximum waveform peak as a waveform feature is specified from the components corresponding to waveform peaks appearing in the measurement waveform as obtained in the waveform forming step S31 as shown above.

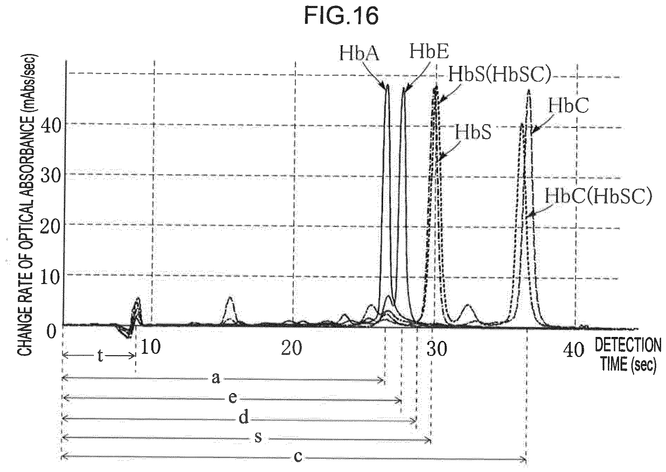

Fig. 16 illustrates the electropherograms of Fig. 11 to Fig. 15 overlaid together. As clearly seen in Fig. 16, the waveform peak arising due to HbC is obtained at a noticeably later detection time than those of the waveform peaks for HbA, HbE, HbD (not illustrated), and HbS. Moreover, while the waveform peaks for HbA, HbE, HbD (not illustrated), and HbS are all close to one another, the detection times become progressively later in this sequence. Namely, providing that the detection times of the waveform peaks for HbA, HbE, HbD, HbS, and HbC are defined as "a", "e", "d", "s", and "c", respectively, and that the interface detection time is defined as "t", it is clear that, as shown in Fig. 16, "a", "e", "d", "s", and "c" are greater than "t", of course, and "c" is larger than "a", "e", "d", and "s".

Specifically, first, the maximum waveform peak as the waveform peak with the greatest area is identified in the measurement waveform obtained in the waveform forming step. As described above, the area of a waveform peak may be defined as an area surrounded by the waveform peak between the bottoms (see Fig. 12) appearing before and after the peak top.

Next, the second waveform peak as the waveform peak having the second greatest area is specified in the same measurement waveform. Note that as illustrated in Fig. 11 to Fig. 14, although the maximum waveform peak is clear in the case of homozygotes, some very small waveform peaks are also seen. Then, the second waveform peak may be considered to be present as long as it occupies more than a predetermined proportion to the total area of the measurement waveform. Otherwise, providing that no second waveform peak is present, and a homozygous hemoglobin component is identified using the maximum waveform peak only.

Component Identification Step S34

In this step, the component is identified based on a comparison of the maximum-peak detection time at which the maximum waveform peak is detected, which is specified in the waveform peak specification step S33 (and the second-peak detection time at which the second waveform peak is detected if the second waveform peak is present), to the interface detection time. In the following description, identification of the hemoglobin component is explained in a specimen containing at least either an HbC component apparently distinguishable from other hemoglobin components or an HbA component actually appearing most frequently.

In measurement and analysis using the disposable analysis chip 2, it is demanded to obtain an accurate analysis result from only a single trial of analysis, without performing a correction that is possible with plural trials of measurements, even when the detection times for the same component would fluctuate as a result of differences between individual analysis chips 2. When identifying hemoglobin types based on the peak time (i.e. detection time) of a maximum waveform peak in a single trial of analysis, sometimes it might not be possible to accurately determine the component, resulting in incorrect analysis results, if there is a large degree of fluctuation in the detection times of the components to be analyzed.

In consideration of such issues, it is possible to identify hemoglobin components corresponding to the waveform peaks without influence by the fluctuations in the detection time caused by differences between individual analysis chips, relativizing the detection time of the waveform peak by the interface detection time.

Specifically, first, the detection time corresponding to the peak top of the maximum waveform peak specified in the waveform peak specification step S33 is taken as the maximum-peak detection time (p1).

Next, in cases in which a second waveform peak is specified in the waveform peak specification step S33, the detection time corresponding to the peak top of the second waveform peak is taken as the second-peak detection time (p2).

Then, for example, as illustrated in Fig. 16, it is possible to determine that the maximum waveform peak is derived from an HbC component if the ratio of the maximum-peak detection time (p1) or the second-peak detection time (p2), to the interface detection time (t) (in other words, a value obtained by dividing the maximum-peak detection time or the second-peak detection time by the interface detection time) is greater than a predetermined value, based on the fact that the detection time (c) of the waveform peak derived from an HbC component is later than the detection times for other waveform peaks ("a", "e", "d", or "s").

It is preferable for the predetermined value to be a value "x" satisfying the following relationship, based on both the greatest detection time other than for an HbC component (the detection time "s" of the waveform peak for an HbS component in Fig. 16), and the detection time "c" for the waveform peak for HbC:

$$(s/t) < x < (c/t).$$

(1) Cases in which an HbC component is present

A maximum waveform peak or a second waveform peak can be identified to be derived from an HbC component if either of the following relationships is true for the maximum-peak detection time (p1) or the second-peak detection time (p2), respectively:

$$x < (p1/t) \text{ or}$$

x < (p2/t).

(1-1) Cases in which a second waveform peak is absent

In such cases, the specimen type is identified as an HbCC specimen when only a maximum waveform peak is specified, and a second waveform peak is not specified..

(1-2) Cases in which a second waveform peak is present

In cases in which both a maximum waveform peak and a second waveform peak are present, the specimen type may be considered to be heterozygous including an HbC component, namely to be either of an HbCA specimen, an HbCE specimen, an HbCD specimen, an HbCS specimen, an HbSC specimen, an HbDC specimen, an HbEC specimen, or an HbAC specimen.

Then, the components corresponding to the maximum waveform peak and the second waveform peak can be identified based on a value "y" defined as follows according to a comparison between the maximum-peak detection time (p1), the second-peak detection time (p2), and the interface detection time (t), thereby enabling the specimen type to be identified:

$$y = (p1 - t) / (p2 - t).$$

For example, if $0.64 \leq y < 0.67$, the specimen is identified to be an HbCA specimen. It is also possible for other heterozygous specimens to be identified by setting these values appropriately.

(2) Cases in which an HbC component is absent

If $(p1/t) \leq x$, and $(p2/t) \leq x$, neither the maximum waveform peak nor the second waveform peak are due to an HbC component specimen. Thus, the maximum waveform peak and the second waveform peak may both be thought to be derived from either an HbA, HbE, HbD, or HbS component. Note that as described above, hemoglobin components other than HbA components appear only infrequently, and so in cases in which the maximum waveform peak is determined not to be derived from an HbC component, the maximum waveform peak is determined to be derived from an HbA component.

(2-1) Cases in which a second waveform peak is absent

In cases in which a second waveform peak is absent, as described above, the maximum waveform peak is determined to be derived from an HbA component, and so the specimen is determined to be an HbAA specimen.

Note that when only a maximum waveform peak is present, although such cases are infrequent, it is possible that the maximum waveform peak may reflect either an HbEE specimen, an HbDD specimen, or an HbSS specimen rather than a homozygous HbAA specimen identified as described above. In such cases, it is possible to use the maximum-peak detection time (p1) and the interface detection time (t) to determine if the specimen is either of these homozygous specimens.

For example, in cases in which a waveform peak thought to be derived from an HbA2 component is present in a range between a lower value of $\{(p1 - t) \times 0.94 + t\}$ and an upper value of $\{(p1 - t) \times 0.97 + t\}$, there is a suspicion that the specimen may be an HbEE specimen.

Note that it is possible to identify that the specimen is an HbAA specimen by utilizing the waveform peak of an HbA1 component, and the detection time thereof.

(2-2) Cases in which a second waveform peak is present

In cases in which, for example, a second waveform peak is specified in addition to a maximum waveform peak, the specimen may be thought to be a heterozygous specimen that does not include an HbC component but does include an HbA component, namely either an HbSA specimen, an HbDA specimen, an HbEA specimen, an HbAE specimen, an HbAD specimen, or an HbAS specimen.

In such cases, the value "y" described above can be employed to identify components corresponding to the maximum waveform peak and the second waveform peak, and thereby the specimen type can be identified.

For example, if $0.81 \leq y < 0.87$, the specimen is identified to be an HbSA specimen. It is also possible for other heterozygous specimens to be identified by setting these values appropriately. Note that sometimes the value "y" may fall within the same range, or partially overlap, even though the specimen types are different. In such cases, it is preferable to note that the specimen is suspected to be either of such specimens.

Note that in the above explanation, identification of the specimen type assumes that the specimen is heterozygous with an HbA component in cases in which there is no waveform peak derived from an HbC component, and a second waveform peak is present. However, there is actually a possibility that the specimen is a heterozygous specimen in which the maximum waveform peak and the second waveform peak are derived from neither an HbA component nor an HbC component. It is also possible for such heterozygous combinations having neither HbA components nor HbC components to be identified using the value "y" described above.

In such situations, it is conceivable that there may be cases in which it is difficult to make a determination using only the value "y". In such cases, the measurement waveform may be examined and auxiliary information for the identification with reference to the position and area of a third waveform peak. Conceivable components utilizing a third waveform peak include HbA1c, HbA2, and HbF. Moreover, it is preferable to note that the specimen is suspected to be either of hemoglobin specimens.

Regarding the inequality signs used to indicate numerical ranges in the inequalities in the above explanation, whether a less-than-or-equal-to sign (≤) is applied to the lower limit or the upper limit may be decided as appropriate in consideration of the measurement conditions.

Note that it is possible for the disposable analysis chips 2 employed in the present analysis method to have variations between different lots during manufacture. Since analysis chips 2 of an identical lot have substantially the same dimensions in every part, and so equivalent tendencies are seen in fluctuations in waveform peak detection times during analysis. Accordingly, it is preferable to perform separation and measurements using capillary electrophoresis in advance for each manufacturing lot, and to collect information as to variations among different lots, such as tendencies for fluctuation in waveform peak detection times. During sample analysis, prior to the component identification step (S34), a detection time correction step may be performed based on the information as to difference among different lots for the analysis chips 2. Such an analysis method enables a drop in analysis accuracy to be suppressed even when variation is present between analysis chips 2 of different lots.

Detailed explanation has been given regarding an embodiment of the present application. However, the analysis method according to the present application is not limited to the embodiment described above. Various design modifications within the scope of the appended claims may be applied to the specific configurations of the respective elements of the analysis method according to the present application.

**Claims**

1. An analysis method for analyzing a sample (Sa) using capillary electrophoresis by applying a voltage to a sample solution introduced to a micro flow path, performing separation analysis for a component contained in the sample solution, and measuring an optical measurement value at a measurement section of the micro flow path corresponding to an elapsed time after a start of measurement, the analysis method comprising:

   a waveform forming step (S31) of forming a waveform related to the optical measurement value corresponding to the elapsed time since the start of measurement;
   an interface detection step (S32) of determining an interface detection time when an interface between the sample solution and a migration liquid (Lm) reaches a predetermined measurement position in the micro flow path, based on the optical measurement value;
   a waveform peak specification step (S33) of specifying waveform peaks appearing in the waveform and of identifying a maximum waveform peak which is the largest waveform peak out of the waveform peaks;
   **characterized by**
   a component identification step (S34) of identifying a component contained in the sample solution, said component corresponding to the maximum waveform peak, based on a maximum-peak detection time which is the elapsed time corresponding to the maximum waveform peak, as well as on the interface detection time.

2. The analysis method of claim 1, wherein, in the interface detection step (S32), the interface detection time is determined based on a change in the optical measurement value when the interface reaches the predetermined measurement position.

3. The analysis method of claim 1 or claim 2, wherein: the optical measurement value is an optical absorbance.

4. The analysis method of claim 3, wherein in the component identification step (S34), the component corresponding to the maximum waveform peak is identified based on a comparison of the maximum-peak detection time to the interface detection time.

5. The analysis method of claim 3, wherein in the component identification step (S34), components corresponding to the maximum waveform peak and a second waveform peak are identified based on a comparison between:

   the maximum-peak detection time,
   a second-peak detection time, which is the elapsed time corresponding to the second waveform peak which is the second largest waveform peak out of the waveform peaks specified in the waveform peak specification step, and
   the interface detection time.

6. The analysis method of claim 5, wherein in the component identification step (S34), components corresponding to the maximum waveform peak and the second waveform peak are identified based on a ratio of a difference between the maximum-peak detection time and the interface detection time, to a difference between the second-peak detection time and the interface detection time.

7. The analysis method of any one of claims 1 to claim 6, wherein the sample solution is a solution containing blood as a sample (Sa), and the component is hemoglobin.

8. The analysis method of any one of claims 1 to claim 7, wherein a disposable analysis chip (2) that includes the micro flow path is employed.

**Patentansprüche**

1. Analyseverfahren zum Analysieren einer Probe (Sa) unter Verwendung von Kapillarelektrophorese durch Anlegen einer Spannung an eine in einen Mikroströmungsweg eingeführte Probenlösung, Durchführen einer Trennanalyse für eine in der Probenlösung enthaltenen Komponente und Messen eines optischen Messwerts an einem Messabschnitt des Mikroströmungswegs entsprechend einer verstrichenen Zeit nach einem Messbeginn, wobei das Analyseverfahren umfasst:

einen Wellenformbildungsschritt (S31) zum Bilden einer Wellenform, die sich auf den optischen Messwert bezieht, der der seit dem Messbeginn verstrichenen Zeit entspricht;
einen Grenzflächendetektionsschritt (S32) zum Bestimmen einer Grenzflächendetektionszeit, wenn, basierend auf dem optischen Messwert, eine Grenzfläche zwischen der Probenlösung und einer Migrationsflüssigkeit (Lm) eine vorbestimmte Messposition im Mikroströmungsweg erreicht;
einen Wellenform-Peak-Spezifikationsschritt (S33) zum Spezifizieren der in der Wellenform auftretenden Wellenform-Peaks und zum Identifizieren eines maximalen Wellenform-Peaks, der der größte Wellenform-Peak unter den Wellenform-Peaks ist;
**gekennzeichnet durch**
einen Komponenten-Identifikationsschritt (S34) zum Identifizieren einer in der Probenlösung enthaltenen Komponente, wobei die Komponente, die auf der Grundlage einer Wellenform-Peak-Detektionszeit, die die verstrichene Zeit ist, die dem maximalen Wellenform-Peak entspricht, sowie auf der Grenzflächendetektionszeit dem maximalen Wellenform-Peak entspricht.

2. Analyseverfahren nach Anspruch 1, wobei in dem Grenzflächendetektionsschritt (S32) die Grenzflächendetektionszeit auf der Grundlage einer Änderung des optischen Messwerts bestimmt wird, wenn die Grenzfläche die vorbestimmte Messposition erreicht.

3. Analyseverfahren nach Anspruch 1 oder Anspruch 2, wobei:
der optische Messwert eine optische Absorbanz ist.

4. Analyseverfahren nach Anspruch 3, wobei in dem Komponenten-Identifikationsschritt (S34) die Komponente, die dem maximalen Wellenform-Peak entspricht, auf der Grundlage eines Vergleichs der Maximal-Peak-Detektionszeit mit der Grenzflächendetektionszeit identifiziert wird.

5. Analyseverfahren nach Anspruch 3, wobei in dem Komponenten-Identifikationsschritt (S34) Komponenten, die dem maximalen Wellenform-Peak und einem zweiten Wellenform-Peak entsprechen, auf der Grundlage eines Vergleichs zwischen:

der Maximal-Peak-Detektionszeit,
einer zweiten Peak-Detektionszeit, die die verstrichene Zeit ist, die dem zweiten Wellenform-Peak entspricht, der der zweitgrößte Wellenform-Peak von den Wellenform-Peaks ist, die in dem Wellenform-Peak-Spezifikationsschritt spezifiziert werden, und
der Grenzflächendetektionszeit identifiziert werden.

6. Analyseverfahren nach Anspruch 5, wobei in dem Komponenten-Identifikationsschritt (S34) Komponenten, die dem maximalen Wellenform-Peak und dem zweiten Wellenform-Peak entsprechen, auf der Grundlage eines Verhältnisses einer Differenz zwischen der Maximal-Peak-Detektionszeit und der Grenzflächendetektionszeit zu einer Differenz zwischen der Detektionszeit des zweiten Peaks und der Grenzflächendetektionszeit identifiziert werden.

7. Analyseverfahren nach einem der Ansprüche 1 bis Anspruch 6, wobei die Probenlösung eine Lösung ist, die Blut als eine Probe (Sa) enthält, und die Komponente Hämoglobin ist.

8. Analyseverfahren nach einem der Ansprüche 1 bis Anspruch 7, wobei ein Einweg-Analyse-Chip (2), der den Mikroströmungsweg einschließt, eingesetzt wird.

**Revendications**

1. Procédé d'analyse pour analyser un échantillon (Sa) en utilisant l'électrophorèse capillaire par application d'une tension à une solution échantillon introduite dans une micro-voie d'écoulement, réalisation d'une analyse de séparation pour un composant contenu dans la solution échantillon et mesure d'une valeur de mesure optique à une section de mesure de la micro-voie d'écoulement correspondant à un temps écoulé après un début de mesure, le procédé d'analyse comprenant :

une étape de formation de forme d'ondes (S31) pour former une forme d'ondes relative à la valeur de mesure optique correspondant au temps écoulé depuis le début de mesure ;

une étape de détection d'interface (S32) pour déterminer un temps de détection d'interface où une interface entre la solution échantillon et un liquide de migration (Lm) atteint une position de mesure prédéterminée dans la micro-voie d'écoulement, sur la base de la valeur de mesure optique ;

une étape de spécification de crête de forme d'ondes (S33) pour spécifier des crêtes de forme d'ondes apparaissant dans la forme d'ondes et pour identifier une crête maximale de forme d'ondes qui est la crête de forme d'ondes la plus grande parmi les crêtes de forme d'ondes ; **caractérisé par**

une étape d'identification de composant (S34) pour identifier un composant contenu dans la solution échantillon, ledit composant correspondant à la crête maximale de forme d'ondes, sur la base d'un temps de détection de crête maximale qui est le temps écoulé correspondant à la crête maximale de forme d'ondes, ainsi que du temps de détection d'interface.

2. Procédé d'analyse selon la revendication 1, dans lequel, à l'étape de détection d'interface (S32), le temps de détection d'interface est déterminé sur la base d'un changement dans la valeur de mesure optique lorsque l'interface atteint la position de mesure prédéterminée.

3. Procédé d'analyse selon la revendication 1 ou la revendication 2, dans lequel :
la valeur de mesure optique est une absorbance optique.

4. Procédé d'analyse selon la revendication 3, dans lequel, à l'étape d'identification de composant (S34), le composant correspondant à la crête maximale de forme d'ondes est identifié sur la base d'une comparaison entre le temps de détection de crête maximale et le temps de détection d'interface.

5. Procédé d'analyse selon la revendication 3, dans lequel, à l'étape d'identification de composant (S34), des composants correspondant à la crête maximale de forme d'ondes et à une seconde crête de forme d'ondes sont identifiés sur la base d'une comparaison entre :

le temps de détection de crête maximale, un temps de détection de seconde crête, qui est le temps écoulé correspondant à la seconde crête de forme d'ondes qui est la seconde crête de forme d'ondes la plus grande parmi les crêtes de forme d'ondes spécifiées à l'étape de spécification de crête de forme d'ondes, et le temps de détection d'interface.

6. Procédé d'analyse selon la revendication 5, dans lequel, à l'étape d'identification de composant (S34), des composants correspondant à la crête maximale de forme d'ondes et à la seconde crête de forme d'ondes sont identifiés sur la base d'un rapport d'une différence entre le temps de détection de crête maximale et le temps de détection d'interface, sur une différence entre le temps de détection de seconde crête et le temps de détection d'interface.

7. Procédé d'analyse selon l'une quelconque des revendications 1 à 6, dans lequel la solution échantillon est une solution contenant du sang comme échantillon (Sa) et le composant est l'hémoglobine.

8. Procédé d'analyse selon l'une quelconque des revendications 1 à 7, dans lequel une puce d'analyse jetable (2) qui comprend la micro-voie d'écoulement est utilisée.

13

# FIG.1

# FIG.2

FIG.3

# FIG.4

```
                                              ⌒ S1
┌─────────────────────────────────┐
│        PREPARATION STEP          │
└─────────────────────────────────┘
                 │
                 ▼
                                              ⌒ S2
┌─────────────────────────────────┐
│         ELECTROPHORESIS          │
│              STEP                │
└─────────────────────────────────┘
                 │
                 ▼
                                              ⌒ S3
┌─────────────────────────────────┐
│          ANALYSIS STEP           │
└─────────────────────────────────┘
```

# FIG.5

SAMPLE
COLLECTING STEP ⌐S11

MIXING STEP ⌐S12

MIGRATION LIQUID
FILLING STEP ⌐S13

INTRODUCTION STEP ⌐S14

FIG.6

# FIG.7

# FIG.8

EP 3 477 292 B1

FIG.9

22

FIG.10

WAVEFORM
FORMING STEP    S31

INTERFACE
DETECTION STEP    S32

WAVEFORM PEAK SPECIFICATION STEP
(WAVEFORM FEATURE SPECIFICATION STEP)    S33

COMPONENT
IDENTIFICATION STEP    S34

# FIG.11

FIG.12

FIG.13

FIG.14

FIG.15

## FIG.16

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP S6073458 A
- JP H11337521 A
- JP 2016057289 A